# EUROPEAN PATENT APPLICATION

(11) **EP 3 123 965 A1**
(43) Date of publication of application: **01.02.2017**
(21) Application number: 14887039.7
(22) Date of filing: 25.06.2014
(51) Int. Cl.: A61B 17/3205

(54) **PROTECTION DEVICE FOR PREVENTING SPREAD AND METASTASIS OF TUMOURS IN LAPAROSCOPIC SURGERY**

(30) Priority: 28.03.2014 CN 201410121274
(71) Applicant: Ling, Bin, Chaoyang District, Beijing 100029 (CN); Ling, Andong, Chaoyang District, Beijing 100029 (CN)
(72) Inventor: Ling, Bin, Chaoyang District, Beijing 100029 (CN); Ling, Andong, Chaoyang District, Beijing 100029 (CN)
(74) Representative: Gulde & Partner
(86) International application number: PCT/CN2014/000619
(87) International publication number: WO 2015/143589

(57) **Abstract**

Disclosed in the invention is a protection device for preventing tumor dissemination and tumor metastasis in a laparoscopic surgery which comprises a soft bag (1) for containing a resected tumor during the surgery provided in the abdominal cavity through a puncture in the abdominal wall, and a sealing thread (2) provided around the mouth of the soft bag (1). The sealing cable (2) is tightened and ligated so that the mouth of the soft bag (1) is closed in the pelvic cavity or the abdominal cavity; or the closed mouth of the soft bag (1) can be stretched out of the abdominal cavity through the puncture in the abdominal wall via the sealing thread (2). An instrument can be put in the soft bag in the abdominal cavity through the mouth of the soft bag (1) outside the abdominal cavity, or the instrument can be put into the soft bag in the abdominal cavity through a conduit shaped structure for a breaking operation (3) or a conduit shaped structure for an auxiliary operation (4) and the laparoscope can be put into for observation through a conduit shaped structure for peering (5). The protection device can serve as an auxiliary device in the laparoscopy surgery, in the closed abdominal cavity, a tumor tissue sample is put into the relatively closed protection device, broken and taken out under observation of the laparoscope, avoiding iatrogenic dispersion of a malignant tumor.

## Description

### Technical field

The present invention relates to a surgery auxiliary device, more specifically to a protection device for preventing tumor dissemination and tumor metastasis in a laparoscopic surgery.

### Background

The laparoscope surgery is characterized in that an incision in the abdominal wall is small and hurt and pain from a large incision in the abdominal wall in a traditional surgery are changed, so that it is known as "keyhole" surgery. The volume of tumor resected in the pelvic cavity or the abdominal cavity in the surgery is often large and it is difficult to take out the tumor through a puncture in the abdominal cavity, and in clinical practice, the tumor is needed to be broken by a mastax in the abdominal cavity to be taken out. However, tumor debris generated in the breaking process can easily cause the tumor to disseminate and implant in the pelvic cavity or the abdominal cavity, seriously threatening a patient's life.

### Summary

The invention provides a protection device for preventing tumor dissemination and tumor metastasis in a laparoscopic surgery in order to avoid the existing shortcomings in the prior art as above, wherein tumor tissues resected in the abdominal cavity are isolated in the closed cavity to be broken, and tumor debris generated in the breaking process are confined in the closed cavity to avoid dissemination and metastasis.

The invention adopts the following technical solutions to solve the technical problem:
A protection device for preventing tumor dissemination and tumor metastasis in a laparoscopic surgery, comprises a soft bag (1) for containing a resected tumor during the surgery provided in the abdominal cavity through a puncture in the abdominal wall, and a sealing cable (2) provided around the mouth of the soft bag (1), the sealing cable (2) being tightened and ligated so as to enable the mouth of the soft bag (1) to be closed, the closed mouth of the soft bag (1) can be stretched out of the abdominal cavity through the puncture in the abdominal wall via the sealing thread (2), and an instrument can be put in the soft bag in the abdominal cavity through the mouth of the soft bag (1) outside the abdominal cavity.

In the protection device for preventing tumor dissemination and tumor metastasis in the laparoscopic surgery, a conduit communicated with the soft bag is provided to the tail area of the soft bag (1), the conduit can be a single (3) or multiple (4 or 5), an external port of the conduit can be maintained outside the abdominal cavity or stretched out of the abdominal cavity through the puncture(s) in the abdominal wall, and the instrument can be put in the soft bag in the abdominal cavity through the external port of the conduit.

In the protection device for preventing tumor dissemination and tumor metastasis in the laparoscopic surgery, the external port of the conduit can be closed and the soft bag (1) is aerated through the external port of the conduit, inflating the soft bag (1).

In the protection device for preventing tumor dissemination and tumor metastasis in the laparoscopic surgery, the external port of the conduit can be positioned into an abdominal wall puncture outfit with a one-way gas valve, the instrument is supported by the abdominal wall puncture outfit; and the one-way gas valve is the one-way gas valve opened towards the soft bag in one direction.

In the protection device for preventing tumor dissemination and tumor metastasis in the laparoscopic surgery, the soft bag (1) is a transparent body, and the instrument which passes through conduit includes a grasper and a mastax.

In the protection device for preventing tumor dissemination and tumor metastasis in the laparoscopic surgery, the soft bag (1) is an opaque body, and the instrument which passes through conduit includes a grasper, a mastax and a laparoscope.

Compared to the prior art, the invention has the advantages as follows.
1. The invention provides a protective measure for preventing tumor dissemination and tumor metastasis based on conventional method for breaking a tumor adopted in the laparoscopic surgery.
2. The application of the invention enables the laparoscope to be put into the soft bag through the conduit to directly observe the tumor breaking process, avoiding broken cutters to injury normal tissues.
3. The application of the invention also enables the laparoscope to observe the tumor breaking process through the transparent soft bag, avoiding broken cutters to injury normal tissues.
4. The application of the invention aerates the soft bag through the conduit in the existing closed inflated abdominal cavity in the laparoscope surgery to form another relatively closed space, greatly facilitating the breaking operation of the tumor tissues.

### Brief description of the drawings

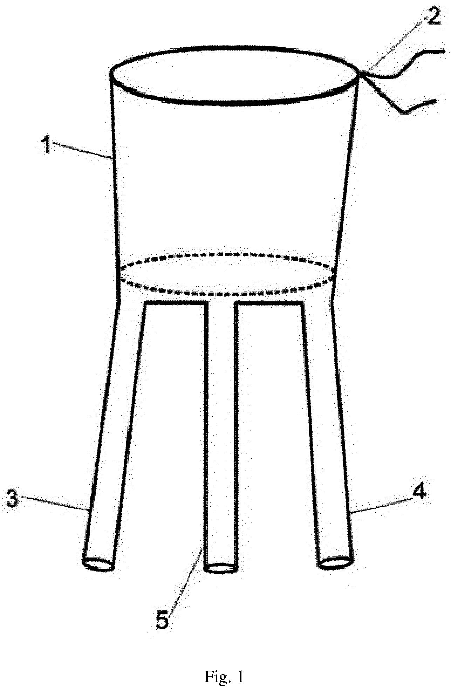

The drawing is the structural representation of the protection device for preventing tumor dissemination and tumor metastasis in the laparoscopic surgery according to the invention.

Reference numbers: 1. soft bag; 2. sealing thread; 3. conduit shaped structure for breaking operation; 4. conduit shaped structure for auxiliary operation; 5. conduit shaped structure for peering.

### Detailed description of the preferred embodiments

Reference to the drawing, the structure of the protection device for preventing tumor dissemination and tumor metastasis in the laparoscopic surgery according to the present embodiment comprises a soft bag (1) for containing a resected tumor during the surgery which is provided in the abdominal cavity through a puncture in the abdominal wall, and a sealing cable (2) provided around the mouth of the soft bag (1), the sealing cable (2) is tightened and ligated after a tumor sample is put into the soft bag (1) so as to enable the mouth of the soft bag (1) to be closed in the pelvic cavity or the abdominal cavity; or the closed mouth of the soft bag (1) can be stretched out of the abdominal cavity through the puncture in the abdominal wall via the sealing thread (2), and a instrument can be put into the soft bag in the abdominal cavity through the mouth of the soft bag (1) outside the abdominal cavity.

A conduit communicated with the soft bag is provided at the tail area of the soft bag (1), and an external port of the conduit can be maintained outside the abdominal cavity or stretched out of the abdominal cavity through other puncture in the abdominal wall, and the instrument can be put into the soft bag in the abdominal cavity through the external port of the conduit. An abdominal wall puncture outfit with a one-way gas valve is provided in the external port of the conduit and the soft bag (1) is aerated through the external port of the conduit, inflating the soft bag (1). The instrument is supported by the abdominal wall puncture outfit; and the one-way gas valve is the one-way gas valve opened towards the soft bag in one direction.

In specific implementation, the soft bag (1) can be a transparent body, and the instrument positioned in the soft bag includes a grasper and a mastax. For this structure, the laparoscope is positioned in the outside of the transparent body to observe the operation. The soft bag (1) can also be an opaque body, and the instrument includes a grasper, a mastax and a laparoscope. For this structure, the laparoscope is positioned in the interior of the opaque body to observe the operation.

### Operation process:

The soft bag is placed into the abdominal cavity through the puncture in the abdominal wall after fold or roll-up, the resected tumor tissue sample is put into the soft bag, the sealing thread is stretched to close the mouth; or the sealing thread is sequentially stretched to enable the mouth of the soft bag to be pulled out of the abdominal cavity through the puncture in the abdominal wall, the instrument is put into the soft bag through the mouth of the soft bag to carry out the breaking operation, and the mouth-closed soft bag is directly pulled out after the breaking operation.

The external port of the conduit is maintained outside the abdominal cavity or stretched out of the abdominal cavity through punctures (3, 4, 5), and the laparoscope or other instrument is put into the soft bag through the conduit for inflation, observation and operation.

The soft bag is aerated to be sufficiency expanded in the abdominal cavity, that is, another relatively closed independent space is constructed in the existing closed inflated abdominal cavity, the laparoscope can be directly put into the independent space through the conduit for observation or be positioned outside the transparent soft bag for observation, the entire process for breaking tumor is completed in the closed independent space, the broken tumor tissues are taken out in the independent space one by one, and the tumor debris tissues generated during the process are enclosed in the independent space.

## Claims

1. A protection device for preventing tumor dissemination and tumor metastasis in a laparoscopic surgery, comprises a soft bag (1) for containing a resected tumor during the surgery provided in the abdominal cavity through a puncture in the abdominal wall, and a sealing thread (2) provided around the mouth of the soft bag (1), the sealing thread (2) being tightened and ligated so that the mouth of the soft bag (1) is closed in the pelvic cavity or the abdominal cavity; or the closed mouth of the soft bag (1) can be stretched out of the abdominal cavity through the puncture in the abdominal wall via the sealing thread (2), and an instrument can be put into the soft bag in the abdominal cavity through the mouth of the soft bag (1) outside the abdominal cavity.

2. The protection device for preventing tumor dissemination and tumor metastasis in the laparoscopic surgery according to claim 1, wherein a conduit communicated with the soft bag is provided at the tail area of the soft bag (1), the conduit can be one (3) or multiple (4 or 5), an external port of the conduit can be maintained outside the abdominal cavity through a single or multiple punctures in the abdominal wall, and a laparoscope or other instrument can be put in the soft bag (1) in the abdominal cavity through the external port of the conduit.

3. The protection device for preventing tumor dissemination and tumor metastasis in the laparoscopic surgery according to claim 2, wherein the external port of the conduit can be closed and the soft bag (1) is aerated through the external port of the conduit, inflating the soft bag (1).

4. The protection device for preventing tumor dissemination and tumor metastasis in the laparoscopic surgery according to claim 3, wherein the external port of the conduit can be positioned into an abdominal wall puncture outfit with an one-way gas valve, the instrument is supported by the abdominal wall puncture outfit; and the one-way gas valve is the one-way gas valve opened towards the soft bag in one direction.

5. The protection device for preventing tumor dissemination and tumor metastasis in the laparoscopic surgery according to claim 1, 2, 3 or 4, wherein the soft bag (1) is a transparent body, and the instrument which passes through the conduit includes a grasper and a mastax.

6. The protection device for preventing tumor dissemination and tumor metastasis in the laparoscopic surgery according to claim 1, 2, 3 or 4, wherein the soft bag (1) is an opaque body, and the instrument which passes through the conduit includes a grasper, a mastax and a laparoscope.
